# FASCICULE DE BREVET EUROPEEN

(11) **EP 1 561 846 B1**
(45) Date de publication et mention de la délivrance du brevet: **11.04.2007**
(21) Numéro de dépôt: 05356020.7
(22) Date de dépôt: 04.02.2005
(51) Int. Cl.: D04B 1/18, D04B 1/24, A41D 13/05, A61F 13/06

(54) **Tricot élastique et article de contention**
Elastische Strickware und Artikel mit Stützwirkung
Elastic knitwear and retention article

(30) Priorité: 06.02.2004 FR 0401165
(43) Date de publication de la demande: 10.08.2005
(73) Titulaire: Marcoux Lafay S.A., 42130 Boen sur Lignon (FR)
(72) Inventeur: Marcoux, Jean-Pierre, 42130 Boen sur Lignon (FR)
(74) Mandataire: Delorme, Nicolas

(56) Documents cités:
- EP-A- 0 681 045
- EP-A- 0 989 219
- FR-A- 2 633 512
- FR-A- 2 781 816
- GB-A- 1 276 826
- US-A- 5 299 435

## Description

La présente invention concerne un tricot élastique et un article de contention à usage sportif ou paramédical.

Dans le cadre d'une pratique sportive ou dans le cas de traumatisme de certains membres, il est bien souvent prescrit le port d'une orthèse de contention. Selon les cas, il peut s'agir de ceinture abdominale, ceinture lombaire, coudière, genouillère etc.

La fonction de ces diverses orthèses est le soutien et le maintien dans une posture correcte de la partie du corps ou du membre présentant une faiblesse ou ayant subi un traumatisme.

A cette fin, une orthèse doit être réalisée dans un matériau présentant une haute élasticité pour assurer le maintien souhaité. A titre d'exemple, une ceinture de soutien lombaire doit être taillée dans un tricot qui offre une force de rappel d'au moins 350CN lorsque qu'il est soumis à un allongement de 30%.

Les tricots qui constituent les articles de contention actuels donnent globalement satisfaction en ce qui concerne le maintien qu'ils procurent. De tels tricots sont par exemple décrits dans FR 2 781816 A.

En revanche, un point très défavorable des tricots actuels tient au fait que lorsqu'ils sont portés à même la peau (ce qui est le mode d'utilisation le plus courant), ils ont tendance à retenir l'humidité. Or, les orthèses de contention ont tendance à favoriser une hypersudation dans les zones directement au contact de la peau. Ce phénomène est amplifié lors de la pratique d'un sport.

Ceci peut alors rendre l'orthèse très inconfortable. Le risque est alors que, en raison de son inconfort, le patient décide d'ôter son orthèse, ce qui bien sûr va à l'encontre de la thérapie préconisée.

L'invention a pour but d'éviter ces inconvénients et propose un procédé de réalisation d'un tricot qui, tout en présentant une forte contention, assure une aération permettant d'évacuer l'humidité due à la transpiration.

A cet effet, l'invention concerne un procédé de réalisation d'un tricot tramé sur un métier rectiligne à deux fontures, caractérisé en ce qu'il comprend au moins cinq opérations successives consistant à :
- réaliser une première rangée de mailles côte 1/1 sur toutes les aiguilles avant et arrière,
- déposer un fil de trame élastique sur la première rangée de maille,
- réaliser une deuxième rangée de mailles côte 1/1 sur toutes les aiguilles avant et arrière,
- réaliser un premier report de mailles d'une aiguille sur deux de la fonture arrière sur la fonture avant avec les aiguilles paires, et
- réaliser un second report de mailles d'une aiguille sur deux de la fonture avant sur le fonture arrière avec les aiguilles impaires de façon à former des jours sur le tricot.

Ainsi l'idée fondamentale à la base de l'invention est de proposer un procédé de fabrication d'un tricot qui combine deux propriétés, à priori, contradictoires à savoir offrir une très forte élasticité pour des applications sportive ou paramédicale toute en garantissant une aération importante par des ouvertures qui constituent autant de points de faiblesse dans le tricot ; le procédé selon l'invention fournit un tricot qui combine d'une part des propriétés d'élasticité qui lui sont conférées par sa trame de fil élastique et d'aération qui lui sont conférées par le report des mailles qui crée des jours dans le tricot.

De façon préférée et pour que les jours soient disposés en quinconce sur le tricot, deux séries de cinq opérations successives alternent :
- un premier cycle ayant un premier report avec les aiguilles paires et un deuxième report avec les aiguilles impaires, et
- un deuxième cycle ayant un premier report avec les aiguilles impaires et un deuxième report avec les aiguilles paires.

De plus, la deuxième rangée de mailles est réalisée avec un guide fil vaniseur alimentant les fontures avec deux fils différents.

Cette disposition permet de mêler un fil élastique ce qui favorise l'élasticité du tricot.

Le tricot obtenu par le procédé comprend :
- une première rangée de mailles constituées d'une côte 1/1,
- une trame réalisée dans un fil élastique,
- une deuxième rangée de mailles constituée d'une côte 1/1,
- les mailles étant reportées transversalement pour former des jours dans le tricot.

De préférence, les jours sont disposés en quinconce, ce qui confère au tricot une esthétique visuelle agréable.

Selon une forme de réalisation de l'invention pour réaliser un tricot de contention :
- la première rangée de mailles est constituée de cinq bouts de microfibre de polyamide de 2/78dtex,
- la trame est constituée de fils élastiques guipés ou non guipés,
- la deuxième rangée de mailles est constituée de cinq fils de microfibre et d'un fil élastique.

L'invention vise également un article de contention, notamment à usage médical, paramédical ou sportif dont, au moins, l'une de ses parties est constituée d'un tricot tel que précédemment décrit.

Selon une forme de réalisation très avantageuse, cet article de contention comprend au moins un élément de renfort tel qu'une baleine en regard duquel est disposé un plateau amortissant constitué par un textile dit tridimensionnel.

Ainsi, cet article de contention qui peut être une ceinture lombaire ou abdominale ne présente aucune rupture de ses propriétés de respirabilité et d'aération, y compris au niveau des éléments de renfort qui, dans l'art antérieur, sont généralement doublés avec une pièce de néoprène, matériau non respirant.

Pour sa bonne compréhension, l'invention est décrite en référence au dessin ci-annexé représentant à titre d'exemple non limitatif une forme de réalisation de tricot élastique selon celle-ci et un article de contention utilisant ce tricot.
**Figure 1** est un graphique de liage de ce tricot.
**Figure 2** représente l'entrelacement des mailles du tricot
**Figures 3 et 4** représentent respectivement les faces endroit et envers d'un article de contention en l'occurrence une ceinture lombaire mettant en oeuvre le tricot selon l'invention,
**Figure 5** est une vue en coupe selon V-V de figure IV.

Ce tricot 1 est réalisé sur une machine à tricoter rectiligne à double fonture selon un procédé présentant cinq opérations successives.

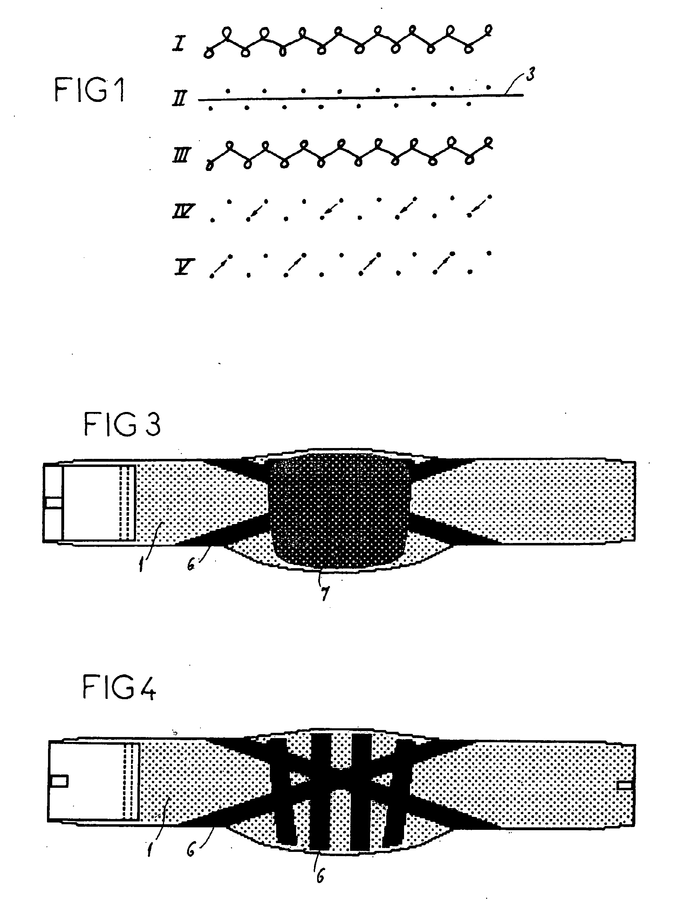

On se référera tout d'abord à la figure 1 qui représente les différentes étapes de réalisation du tricot selon l'invention.

Tout d'abord, on réalise une première rangée I de mailles m1 côte 1/1 en utilisant les aiguilles avant et les aiguilles arrière du métier. Pour cette opération, le guide fil est enfilé de cinq bouts de type microfibres de polyamide constituant le fil 2. Le titrage de chacun de ces bouts est de l'ordre de 2/78 dtex

Dans un deuxième temps II, un fil élastique 3 est déposé sur la première rangée de mailles m1. Dans le mode de réalisation du tricot utilisé pour réaliser un article de contention, ce fil peut être constitué de 23 % d'élasthanne et 77 % de viscose.

Dans un troisième temps, on réalise une côte 1/1 (m3) sur toutes les aiguilles avant et les aiguilles arrière du métier. Le guide fil amenant le fil pour cette opération est un guide fil vaniseur enfilé des cinq bouts de type microfibres de polyamide et d'un fil élastique pouvant présenter 26 % d'élasthanne et 74 % de polyamide.

La grande particularité de l'invention est alors que, à partir de ce tricot présentant une trame élastique :

Dans un quatrième temps, on réalise un report (r1) IV de mailles d'une aiguille sur deux de la fonture arrière sur le fonture avant avec les aiguilles paires.

Dans un cinquième temps, on réalise un report (r2) V de mailles d'une aiguille sur deux de la fonture avant sur le fonture arrière avec les aiguilles impaires.

Grâce à cette contexture très spécifique, ce tricot 1 présente des jours 5 qui constituent autant de passages d'aération. En alternant le report des mailles, les jours 5 sont disposés en quinconce comme on peut la voir sur la figure 2.

Selon les sélections jacquard des reports de mailles, il est possible de faire varier la disposition des jours 5 dans le tricot. Tout en restant dans le cadre de l'invention, le tricot pourrait présenter des jours 5 disposés en colonne, en chevron, etc. Il doit être précisé que la disposition en quinconce de jours 5 s'avère être la disposition optimale quant à l'effet d'aération procuré par le tricot.

La présence de ces jours 5 est donc particulièrement avantageuse pour la réalisation d'articles de contention ; ce tricot procure une réelle aération et permet à l'humidité provenant de la sudation d'un utilisateur de s'échapper par les jours 5 dont le tricot est pourvu.

En dépit de la présence de ces jours, le tricot selon l'invention présente un remarquable maintien grâce à la trame de fil fortement élastique.

Des tests ont ainsi montré que des éprouvettes découpées dans un tricot 1 tel que celui décrit précédemment ayant des dimensions de 10 x 5 cm présente une force de rappel comprise entre 350 et 470 CN lorsqu'elles sont soumises à un allongement de 30 %.

Il peut être envisagé d'utiliser des jauges 7, 8, 10, 12, 14, 16 en utilisant des fils de tricotage et de tramage adaptés, pour obtenir un bon compromis entre épaisseur et ajourage, selon l'usage envisagé du tricot.

Les figures 3 et 4 représentent une ceinture lombaire mettant en oeuvre le tricot 1. Comme on peut le voir, cette ceinture présente à la fois des zones constituées du tricot tel que précédemment défini. En revanche, il est également prévu dans cette ceinture des mailles pleines 6 classiques qui permettent d'occulter des baleines 8 de renfort ou de fournir un maintien supplémentaire dans des zones où cela est nécessaire. On note, par ailleurs, que cette ceinture ne présente pas de couture ce qui la rend particulièrement agréable à porter.

Il est également prévu un plateau 7 rapporté sur la face de la ceinture destinée à venir en contact du porteur.

Le plateau a, comme caractéristique spécifique, d'être constitué d'un textile dit tridimensionnel. Le plateau 7, qui a pour objet d'amortir le contact entre l'utilisateur et les baleines 8 de renfort, est réalisé dans un textile tridimensionnel. Par textile tridimensionnel, on entend un textile constitué de deux nappes maintenues à distance l'une de l'autre par un réseau de fibres. Ce textile assure donc une très grande respirabilité et il ne retient pas l'humidité.

Le plateau spécifique a pour extrême avantage d'offrir une ceinture de contention qui ne présente aucune interruption dans sa propriété de respirabilité et un effet d'hyperventilation.

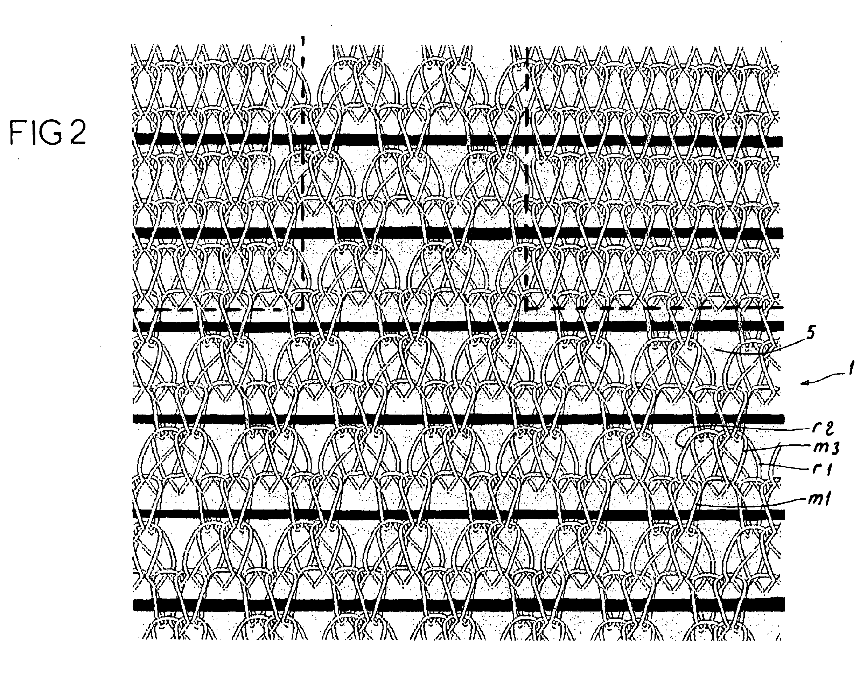

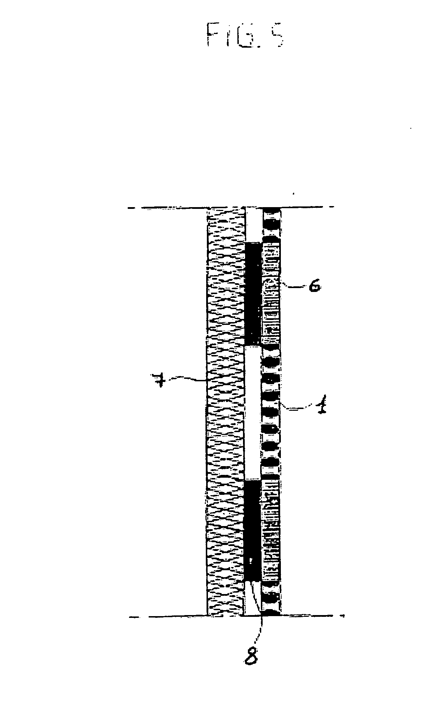

La ceinture, telle que l'on peut la voir sur les figures 3 et 4, ne présente donc aucune discontinuité dans ses propriétés de respirabilité, perméabilité et d'aération, comme le montre la vue en coupe de la figure 5. Ceci la rend, particulièrement agréable à porter.

Bien entendu, l'invention n'est pas limitée aux formes de réalisation décrites ci-dessus à titre d'exemples non limitatifs mais elle embrasse au contraire toutes les variantes de réalisation. Ainsi il est possible d'envisager d'employer des fils constitué de matière autres que celles précédemment décrites. Le tricot selon l'invention peut, par ailleurs, être employé pour réaliser tout type d'article de contention, à savoir genouillère, coudière, ceinture abdominale... En outre, si dans l'exemple représenté, le textile tridimensionnel d'amortissement présente la forme d'un plateau spécialement adapté à une ceinture, il pourrait prendre toute autre forme pour être adapté à d'autres articles de contention.

## Revendications

1. Procédé de réalisation d'un tricot (1) tramé sur un métier rectiligne à deux fontures présentant deux séries d'aiguilles avant et arrière, **caractérisé en ce qu**'il comprend au moins cinq opérations successives consistant à :
- réaliser une première rangée (1) de mailles (m1) côte 1/1 sur toutes les aiguilles avant et arrière,
- déposer (II) un fil de trame (3) élastique sur la première rangée de maille (m1),
- réaliser une deuxième rangée (III) de mailles (m3) côte 1/1 sur toutes les aiguilles avant et arrière,
- réaliser un premier report (IV) (r1) de mailles d'une aiguille sur deux de la fonture arrière sur la fonture avant avec les aiguilles paires, et
- réaliser un second report (V) (r2) de mailles d'une aiguille sur deux de la fonture avant sur le fonture arrière avec les aiguilles impaires de façon à former des jours (5) sur le tricot (1).

2. Procédé selon la revendication 1, **caractérisé en ce que** deux séries de cinq chutes successives comprennent respectivement :
- un premier cycle ayant un premier report (r1) réalisé avec les aiguilles paires et un deuxième report (r2) réalisé avec les aiguilles impaires, et
- un deuxième cycle ayant un premier report (r1) réalisé avec les aiguilles impaires et un deuxième report (r2) réalisé avec les aiguilles paires, de façon à former toutes les cinq chutes des jours (5) disposés en quinconce sur le tricot.

3. Procédé selon la revendication 1 ou la revendication 2, **caractérisé en ce que** la deuxième rangée (III) de mailles est réalisée avec un guide fil vaniseur alimentant les fontures avec deux fils différents.

4. Tricot tramé élastique réalisé selon le procédé visé dans les revendications 1 à 3, **caractérisé en ce qu**'il comprend :
- une première rangée de mailles (m1) constituées d'une côte 1/1,
- une trame réalisée dans un fil élastique,
- une deuxième rangée de mailles (m3) constituées d'une côte 1/1,
- les mailles étant reportées transversalement pour former des jours (5) dans le tricot (1).

5. Tricot selon la revendication 4, **caractérisé en ce que** les jours (5) sont disposés en quinconce.

6. Tricot selon l'une des revendications 4 à 5, **caractérisé en ce que** le fil de la première rangée de mailles (1) est constitué de cinq bouts de microfibre de polyamide de 2/78 dtex.

7. Tricot selon la revendication 4, **caractérisé en ce que** la trame est constituée d'un fil élastique guipé ou non guipé.

8. Tricot selon l'une des revendications 4 à 7, **caractérisé en ce que** le fil de la deuxième rangée de mailles est constitué de cinq bouts de microfibres de polyamide de 2/78 dtex et d'un fil élastique.

9. Article de contention notamment à usage médical, paramédical ou sportif, **caractérisé en ce que**, au moins l'une de ses parties est constituée d'un tricot selon l'une des revendications 4 à 8.

10. Article de contention selon la revendication 9, **caractérisé en ce qu**'il comprend au moins un élément de renfort tel qu'une baleine (8) en regard duquel est superposé un plateau (7) amortissant constitué par un textile dit tridimensionnel.

## Claims

1. Method of producing a weft knit (1) on a rectilinear knitting machine with two needle beds having two series of front and rear needles, **characterized in that** it comprises at least five successive operations consisting in:
- producing a first course (I) of 1/1 rib stitches (m1) on all the front and rear needles;
- depositing (II) an elastic weft yarn (3) onto the first row of stitches (ml);
- producing a second course (III) of 1/1 rib stitches (m3) on all the front and rear needles;
- performing a first transfer (IV) (r1) of stitches on one needle in two from the rear needle bed onto the front needle bed with the even needles; and
- performing a second transfer (V) (r2) of stitches on one needle in two from the front needle bed onto the rear needle bed with the odd needles, so as to form apertures (5) in the knit (1).

2. Method according to Claim 1, **characterized in that** two series of five successive feeders comprise, respectively:
- a first cycle having a first transfer (r1) performed with the even needles and a second transfer (r2) performed with the odd needles; and
- a second cycle having a first transfer (r1) performed with the odd needles and a second transfer (r2) performed with the even needles, so as to form, every five feeders, apertures (5) arranged in a staggered fashion in the knit.

3. Method according to Claim 1 or Claim 2, **characterized in that** the second course (III) of stitches is produced with a plating yarn guide feeding the needle beds with two different yarns.

4. Elastic weft knit produced according to the method indicated in Claims 1 to 3, **characterized in that** it comprises:
- a first course of stitches (m1) consisting of a 1/1 rib;
- a weft made in an elastic yarn;
- a second course of stitches (m3) consisting of a 1/1 rib; and
- the stitches being transferred transversely in order to form apertures (5) in the knit (1).

5. Knit according to Claim 4, **characterized in that** the apertures (5) are arranged in a staggered fashion.

6. Knit according to either of Claims 4 and 5, **characterized in that** the yarn of the first course of stitches (I) consists of five 2/78 dtex polyamide microfibre ends.

7. Knit according to Claim 4, **characterized in that** the weft consists of a covered or non-covered elastic yarn.

8. Knit according to one of Claims 4 to 7, **characterized in that** the yarn of the second course of stitches consists of five 2/78 dtex polyamide microfibre ends and an elastic yarn.

9. Support article, especially for medical, paramedical or sports use, **characterized in that** at least one of its parts consists of a knit according to one of Claims 4 to 8.

10. Support article according to Claim 9, **characterized in that** it includes at least one reinforcing element such as a stiffener (8) opposite which a damping plate (7), consisting of a three-dimensional textile, is superposed.

## Patentansprüche

1. Verfahren zur Herstellung einer Schusswirkware (1) auf einer Flachwirkmaschine mit zwei Nadelplatten, die zwei Reihen von Nadeln, eine vordere und eine hintere, aufweisen, **dadurch gekennzeichnet, dass** es mindestens fünf aufeinanderfolgende Vorgänge aufweist, die darin bestehen:
- eine erste Reihe (I) von Maschen (m1) gerippt 1/1 auf allen vorderen und hinteren Nadeln auszuführen,
- einen elastischen Schuss (II) (3) auf die erste Reihe von Maschen (m1) aufzubringen,
- eine zweite Reihe (III) von Maschen (m3) gerippt 1/1 auf allen vorderen und hinteren Nadeln auszuführen,
- einen ersten Übertrag (IV) (r1) von Maschen jeder zweiten Nadel des hinteren Nadelbetts auf das vordere Nadelbett mit den geradzahligen Nadeln durchzuführen, und
- einen zweiten Übertrag (V) (r2) von Maschen jeder zweiten Nadel des vorderen Nadelbetts auf das hintere Nadelbett mit den ungeradzahligen Nadeln durchzuführen, um Löcher (5) in der Wirkware (1) zu bilden.

2. Verfahren nach Anspruch 1, **dadurch gekennzeichnet, dass** zwei Reihen von fünf aufeinanderfolgenden "Chutes" jeweils aufweisen:
- einen ersten Zyklus mit einem ersten Übertrag (r1), der mit den geradzahligen Nadeln durchgeführt wird, und mit einem zweiten Übertrag (r2), der mit den ungeradzahligen Nadeln durchgeführt wird, und
- einen zweiten Zyklus mit einem ersten Übertrag (r1), der mit den ungeradzahligen Nadeln durchgeführt wird, und mit einem zweiten Übertrag (r2), der mit den geradzahligen Nadeln durchgeführt wird, um alle fünf "Chutes" der Löcher (5) zu formen, die versetzt in der Wirkware angeordnet sind.

3. Verfahren nach Anspruch 1 oder Anspruch 2, **dadurch gekennzeichnet, dass** die zweite Reihe (III) von Maschen mit einer Plattier-Fadenführung durchgeführt wird, die die Nadelbetten mit zwei verschiedenen Fäden speist.

4. Elastische Schusswirkware, die gemäß dem Verfahren der Ansprüche 1 bis 3 hergestellt wird, **dadurch gekennzeichnet, dass** sie aufweist:
- eine erste Reihe von Maschen (m1), die aus einer Rippe 1/1 bestehen,
- einen Schussfaden aus einem elastischen Faden,
- eine zweite Reihe von Maschen (m3), die aus einer Rippe 1/1 bestehen,
- wobei die Maschen quer übertragen werden, um Löcher (5) in der Wirkware (1) zu bilden.

5. Wirkware nach Anspruch 4, **dadurch gekennzeichnet, dass** die Löcher (5) versetzt angeordnet sind.

6. Wirkware nach einem der Ansprüche 4 bis 5, **dadurch gekennzeichnet, dass** der Faden der ersten Maschenreihe (I) aus fünf Polyamid-Mikrofaserfäden mit 2/78 dtex besteht.

7. Wirkware nach Anspruch 4, **dadurch gekennzeichnet, dass** der Schussfaden aus einem umsponnenen oder nicht umsponnenen elastischen Faden besteht.

8. Wirkware nach einem der Ansprüche 4 bis 7, **dadurch gekennzeichnet, dass** der Faden der zweiten Maschenreihe aus fünf Polyamid-Mikrofaserfäden mit 2/78 dtex und aus einem elastischen Faden besteht.

9. Stützartikel, insbesondere zur medizinischen, paramedizinischen oder sportlichen Verwendung, **dadurch gekennzeichnet, dass** mindestens eines seiner Teile aus einer Wirkware nach einem der Ansprüche 4 bis 8 besteht.

10. Stützartikel nach Anspruch 9, **dadurch gekennzeichnet, dass** er mindestens ein Verstärkungselement wie ein Stäbchen (8) aufweist, dem gegenüber eine Dämpfungsplatte (7) überlagert ist, die aus einem so genannten dreidimensionalen Wirkware besteht.
